# EUROPEAN PATENT SPECIFICATION

(11) **EP 1 057 453 B1**
(45) Date of publication and mention of the grant of the patent: **09.06.2004**
(21) Application number: 00111537.7
(22) Date of filing: 30.05.2000
(51) Int. Cl.: A61B 8/02

(54) **Electronic foetal heart simulator**
Elektronischer Fetalherz-Simulator
Simulateur électronique du coeur foetal

(30) Priority: 29.05.1999 GB 9912504
(43) Date of publication of application: 06.12.2000
(73) Proprietor: Viamed Limited, Keighley, West Yorkshire BD20 7DT (GB)
(72) Inventor: Brough, David, Hartlepool TS24 8JP (GB); Scott, Leslie Arthur, Hartlepool TS25 5LD (GB)
(74) Representative: Tomkinson, Alex

(56) References cited:
- US-A- 4 974 461
- US-A- 5 718 227
- COHEN ET AL.: "An experimental rig to simulate fetal heart sounds" EUR. J. OBSTET. GYNECOL.. REPROD. BIOL., vol. 23, 1986, pages 273-278, XP000937506
- HIROSHI KANAI ET AL: "A NEW METHOD TO MEASURE LOCAL VIBRATIONS IN A HEART USING ULTRASOUND" PROCEEDINGS OF THE ANNUAL INTERNATIONAL CONFERENCE OF THE ENGINEERING IN MEDICINE AND BIOLOGY SOCIETY,US,NEW YORK, IEEE, vol. CONF. 13, 31 October 1991 (1991-10-31), pages 131-132, XP000346222

## Description

This invention relates to the electronic foetal heart simulator.

Monitoring of the foetal heart is extremely important both before and during labour, it provides clinicians with essential data relating to the wellbeing of the unborn baby. This data is used as a tool in aiding the clinicians in their decision making process regarding the physiological condition of the baby. The information obtained may indicate amongst other things the necessity for surgical intervention in the form of caesarian section if the baby shows signs of hypoxia.

Ultrasonic foetal heart monitors have evolved to provide this facility and are now used extensively in hospitals throughout the world. This type of monitor uses sound waves in the ultrasonic spectrum to derive the foetal heart rate from the physical heart wall movement. The technique entails directing a beam of ultrasound produced via a transducer through the mothers' abdomen towards the foetal heart. This beam is deflected off the moving heart wall, which causes a doppler shift in frequency. The returning beam is received by the monitor's transducer and presented to the processing electronics of the foetal heart monitor, where the doppler shift is detected, and manipulated to produce a signal corresponding to the foetal heart rate. The heart rate is then displayed on a numeric display and also on some monitors on a paper chart recorder. An important parameter also displayed by the latter is the foetal heart accelerations and decelerations, which provide an indication of foetal distress.

The correct operation of these monitors is of paramount importance and hence a reliable and accurate method for testing them is needed. The monitors currently in use emit an audible signal corresponding to each beat of the foetal heart, but the detection of the foetal heart beat signal when the foetus is within the abdomen of its mother involves the use of sensitive electronics. Many nurses, clinicians and the like simply tap the transducer against their hands to establish whether the monitor produces an audible sound, but such a method of testing the apparatus may damage the electronics and cause the monitor to provide incorrect results during a birth. The consequences of such incorrect operation may be fatal.

The main problem of testing these monitors is the requirement to produce controllable simulated heart wall movements and a means of coupling these movements ultrasonically to the monitor's transducer. It has heretofore not been possible to realistically reproduce signals characterising foetal heart movements which can be readily monitored by a conventional foetal heart monitor.

A technical paper published in the Eur. J. Obstet. Gynaecol. Reprod. Biol., at 23 (1986) 273-280 by Cohen, Timbs, and Dalton discloses a foetal heart simulator comprising a water filled chamber in which a constant water pressure is maintained and underneath which a vibrator, an accelerometer, and a small diaphragm are disposed to produce vibrations through the water chamber thereabove. A membrane, stretched as a result of the water pressure within the chamber, is provided over the upper surface of the chamber, and it is to the outer surface of this membrane that a foetal heart monitor is applied. This arrangement is unsatisfactory because attenuation substantially distorts the vibrations through the water chamber and thus a signal effectively reproducing that measurable from a living foetus cannot be accurately simulated.

It is the object of the present invention to provide a foetal heart simulator which can be effectively coupled to a modern foetal heart monitor which realistically reproduces common and uncommon foetal heart movements.

According to the present invention there is provided a foetal heart simulator comprising an electronically controllable movement transducer connected to signal processing and production means which is capable of reproducing a frequency spectrum similar to that exhibited by the heart of a foetus, characterised in that the transducer is embedded within a gel-based coupling medium enclosed within a casing.

Preferably layers of the gel-based coupling medium are disposed on both sides of the transducer, in particular on both sides of the axis of said transducer which is perpendicular to the axis of physical excitation thereof.

Preferably the signal processing and production means comprises an electronic control system.

Preferably the foetal heart simulator comprises a casing within which the transducer is disposed embedded within the gel-based coupling medium which completely fills the inside of said simulator such that at least an amount of coupling medium separates the transducer from the casing on all sides.

Preferably the signal processing and production means is capable of producing an excitation waveform to drive the transducer which is representative of the physiological response of a foetal heart as is measurable by foetal heart monitors.

Preferably, the signal processing and production means incorporates signal summation means and separate signal production means capable of producing signals representative of different physiological conditions which may be prevalent in an abnormal foetal heart.

In one aspect of the invention, one of the separate signal production means is capable of providing a signal representative of foetal heart movements, while another signal production means is capable of producing signals representative of a physiological phenomenon, for example arrhythmia, hypoxia, muscular difficulties or other condition which may cause distress in a foetus and thus affect the heart rate thereof. The summation of these two separate signals results in a signal which drives the transducer within the simulator in a manner which is representative of a foetal heart in distress.

Preferably the transducer is a piezo-electric transducer.

A specific embodiment of the invention is now provided by way of example with reference to the accompanying diagrams wherein:
Figure 1 shows a schematic representation of a foetal heart simulator according to the invention.
Figure 2 shows a graph of amplitude vs. time for a signal which effectively reproduces the signal received from a living foetal heart.
Figure 3 shows a circuit diagram of the foetal heart prototype drive unit,
Figure 4 shows an analogue reproduction by the circuit of Figure 3 of a waveform of a digitised rudimentary foetal heart signature,
Figure 5 shows an analogue reproduction by the circuit of Figure 3 of a waveform of a digitised negative then positive going step function,
Figure 6 shows an analogue reproduction by the circuit of Figure 3 of a waveform of a digitised single peaked, amplitude modulated envelope,
Figure 7 shows an analogue reproduction by the circuit of Figure 3 of a waveform of a digitised double peaked amplitude modulated envelope, and
Figure 8 shows a further schematic representation a foetal heart simulator.

An electronic foetal heart simulator X consists of a main simulator body (1) with an access hole (2) for connecting cables (3), an electronic movement transducer (4) disposed internally of the simulator and surround by a suitable coupling and embedding material (5) to enable ultrasonic attenuation and coupling.

During operation, a conventional foetal heart monitor (6) electrically connected to a transducer (7) contacts a commercially available coupling medium (8) which is in turn disposed in contact with the outer surface of the main simulator body(1). The monitor (6) causes the emission of ultrasonic waves from the enclosed housing (7) which, after being attenuated by, and passing through the suitable gel-based coupling material (5) in which transducer (4) is embedded, are deflected by the electronic excitation of transducer (4) which is actuated by drive and control electronics (9) to produce a movement within the coupling and embedding material. This movement causes a doppler effect upon the signal transmitted by the foetal heart monitor (6) under test. This signal is then filtered, analysed and displayed by the said foetal heart monitor under test.

It is to be mentioned that the drive electronics (9) may be provided at the base of the simulator body, and either mains or battery powered. It is important to point out that the isolation of the transducer is important and the gel-based coupling material is essential in this regard. A suitable gel is sold by Fellows under the trade name MEMOFLEX, but the applicants have experimented with a wide variety of medical and commercially available gel-based compounds and found that the vast majority of such gel compounds result in a desired response. Indeed commonly available hair gels achieve the desired results and therefore the particular type of the gel-based compound is unimportant, except that the gel chosen should be stable and should not degrade significantly over the expected life of the simulator. Water and alcohol based gels of a low viscosity would therefore be unsuitable because the alcohol or water in the gel compound can evaporate rendering the gel solid or otherwise compromised.

Additonally, it is advantageous if the gel-based compound in which the transducer is embedded has a high degree of elasticity and recovers quickly and repeatably after elastic deformation.

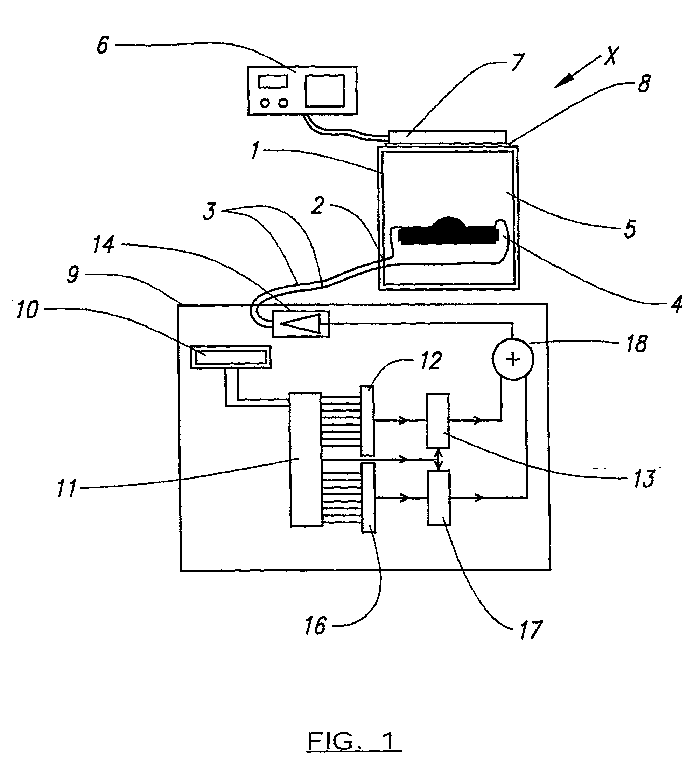

Figure 1 shows a foetal heart monitor (6) being tested by the electronic foetal heart simulator which is connected to accompanying processing and drive electronics (9). The foetal heart monitor produces an ultrasound signal via crystals embedded within the enclosed housing (7). This signal, typically 2Mhz, is coupled via the commercially available medium (8) (which may itself be a gel) to the main simulator body (1). After passing through the main simulator body (1), the signal is attenuated by, and passes through the coupling and embedding material (5). Some of the signal is reflected by the change in medium caused by the presence of the electronic movement transducer (4), and some is reflected by the presence of the main simulator body (1). When the electronic movement transducer (4) is static, the signal received by the foetal heart monitor via the housing (7) contains no Doppler shift component, and hence when the carrier frequency is removed by the foetal heart monitor which indicates the absence of foetal heart information is present.

A complex electronic signal of similar frequency spectrum to that of a foetal pulse is produced by a micro-processor (11) incorporated within the drive and control electronics. This signal is processed in a Digital to Analogue converter (D/A network) (12), the output of which is passed through a controllable signal attenuator (13) to a summation device (18), and ultimately through amplification means (14). The output signal of this amplification means produces movement of the electronic movement transducer (4). This movement causes a doppler effect upon the signal emanating from the housing (7), which when processed by the foetal heart monitor (6) causes said foetal heart monitor (6) to respond by displaying a pulse rate equal to that set up by the micro-processor (11). The processing and drive electronics (9) can also be programmed such that the pulse rate presented to the electronic movement transducer (4) can be altered with respect to time producing an acceleration or deceleration in pulse rate which can confirm the specifications of the foetal heart monitor(6).

An additional signal may be produced by the microprocessor (11) and processed in a secondary D/A network (16), the output of which passes through a further controllable signal attenuator (17), and the output signal of this device may be summed together with the signal from the first controllable signal attenuator (13) by the summation means (18). The invention thus provides a means of simulating all physiological conditions experienced by a foetus. By use of the attenuators (13, 17) and the microprocessor (11) many variations in signal amplitude and shape can be converted into movement by the electronic movement transducer (4), thus the foetal monitor (6) can be made to display information equating to many problems associated with an abnormal foetus.

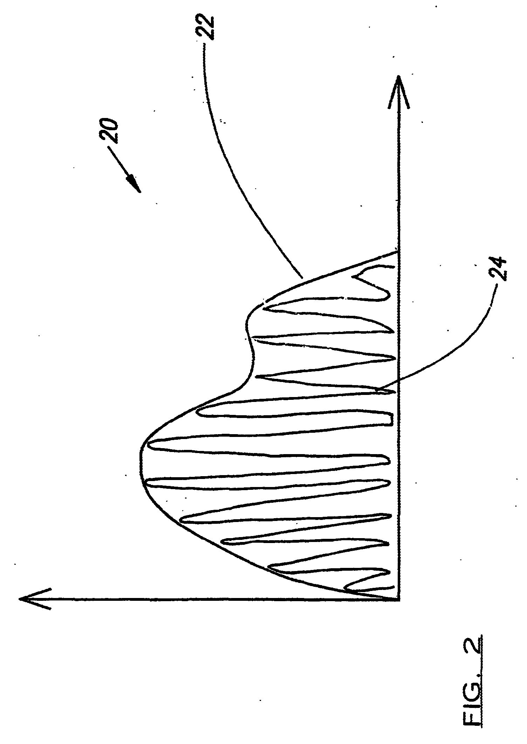

Referring now to Figure 2, there is shown a graph 20 in which two separate signals are shown. In practice only a single signal made up of an amplitude modulated combination of the two signals shown in Figure 2 is sent to the transducer. An envelope signal 22 of low frequency is produced by one of the D/A channels on the microprocessor by storing the waveform is memory provided therewith.

A second, higher frequency signal 24 (of typically up to 500 Hz) is also stored in the memory associated with the microprocessor and can be faithfully reproduced on a second D/A channel. The use of an electronic multiplier within the signal processing circuitry combines the two signals to result in a signal which is effectively the higher frequency signal amplitude modulated by the first lower frequency signal.

In this manner, the lower frequency signal, which is representative of the foetal heart wall movement, can be combined with a higher frequency signal, which can be representative of a wide number of different foetal heart artefacts such as parental tremor, heart valve movement, irregular bloodflow and signals commonly experienced by a foetal heart in distress, and therefore it is possible to effective simulate any of a wide number of foetal heart conditions. It has been discovered by the applicants herefor that it was necessary for the transducer drive signal to contain both the higher frequency component and the lower frequency component for effective and correct simulation of the foetal heart. During experimentation, it was established that without simultaneously combining a high frequency component with the lower frequency component representative of the foetal heart wall movements, the foetal monitor under test registered the heart rate but no audio frequencies characteristic of a heart sound. The combination of the two types of frequency signals thus ensures a faithful simulation of a live foetal heart.

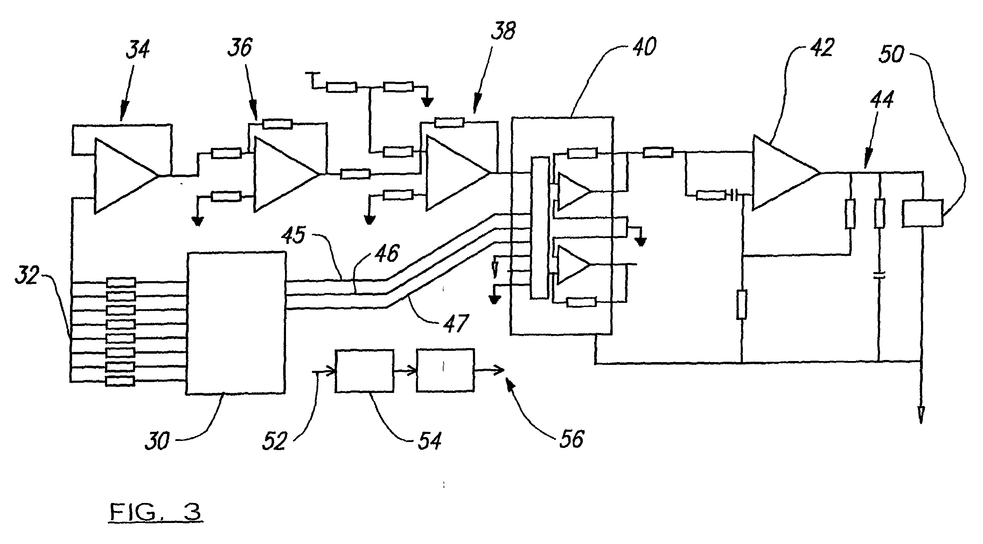

Figure 3 shows a possible electronic circuit for producing the transducer drive signal, and in particular there is provided an 8-bit processor unit 30 having an 8 discrete digital signal outputs which are converted to an analogue signal through a series of operational amplifiers 34, 36, 38. The resulting analogue signal is passed to a signal amplifier 40 whose amplification is controlled by the processor 30 via control lines 45, 46, 47, and the output signal from signal amplifier 40 is fed to the transducer through a further operational amplifier 42 and filter circuitry 44 which removes any superfluous noise resulting from the Digital to Analogue conversion.

The circuit shown in the Figure may be mains powered at 52 and include a transformer 54 to provide a steady voltage output of +/- 15V to the entire circuit at 56. Although not shown in Figure 3, the processor is provided with associated memory to store various different waveforms which may be used to perform basic working tests of the simulator or to provide complex excitation signals to the transducer representative of the complex waveform of a foetal heart in distress.

In many instances, foetal heart monitors are not provided with a chart recorder, and therefore the only means for assessing whether a foetal heart is in distress is by assessing the audible output of the monitor. It is therefore essential that both a low frequency signal be combined with a high frequency signal, whether by the processor 30 or by other components in the driving circuitry. The low frequency signal detected by conventional foetal heart rate monitors is converted to an audible signal so that a clinician or midwife can quickly assess the heart rate, up to a possible maximum in the region of 240 beats per minute. The high frequency signal is provided to give an accurate representation of the waveform of a foetal heart beat, which can include many different artefacts characterising particular distress conditions not immediately recognisable from the audible sounds representing the foetal heart beat. Such artefacts would generally be recognised from a printout on a chart recorder of the foetal heart monitor, or shown on a screen.

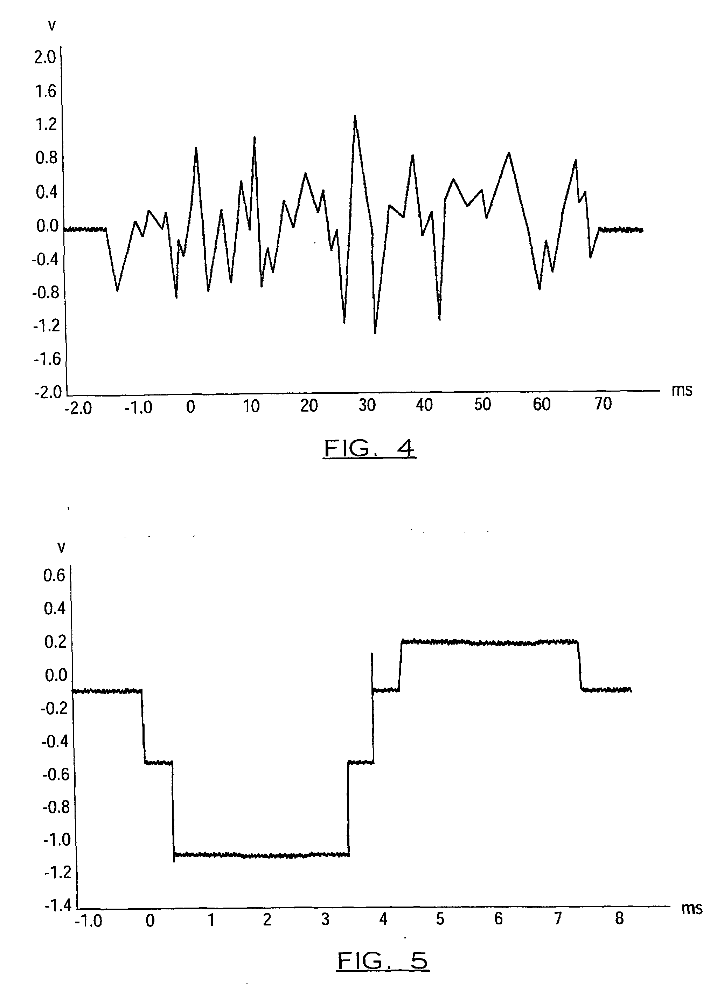

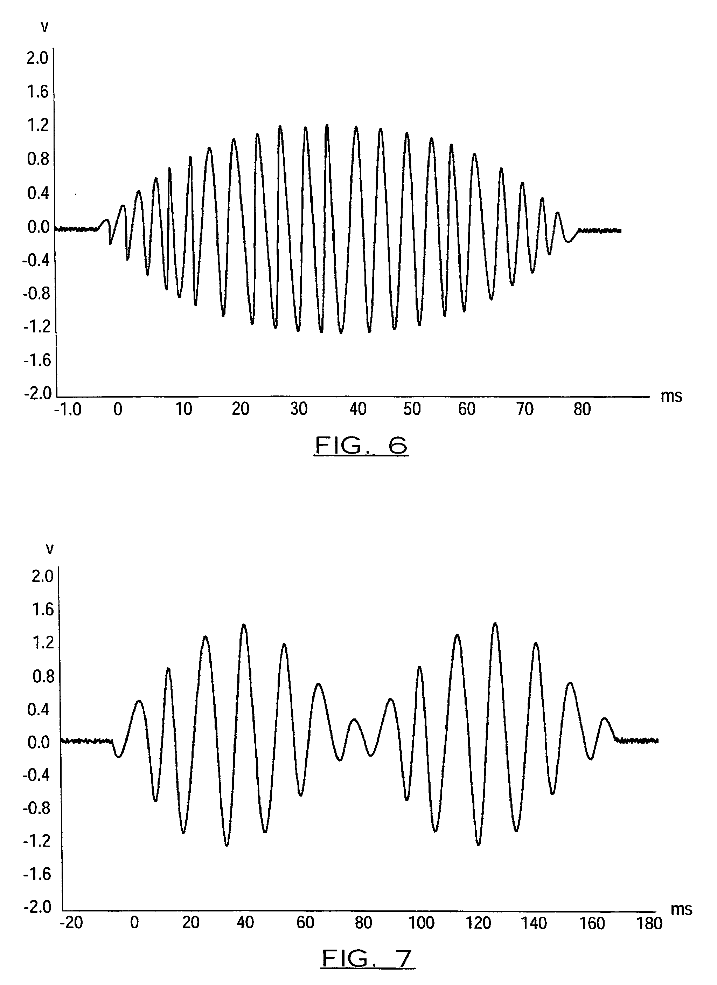

Referring to Figures 4-7, there are shown various different waveforms which are reproduced by the circuitry in Figure 3 from digital information stored in the memory of the processor 30. It will be instantly appreciated that a large number of complex waveforms can be reconstructed, and therefore testing of foetal heart monitors can be particularly comprehensive.

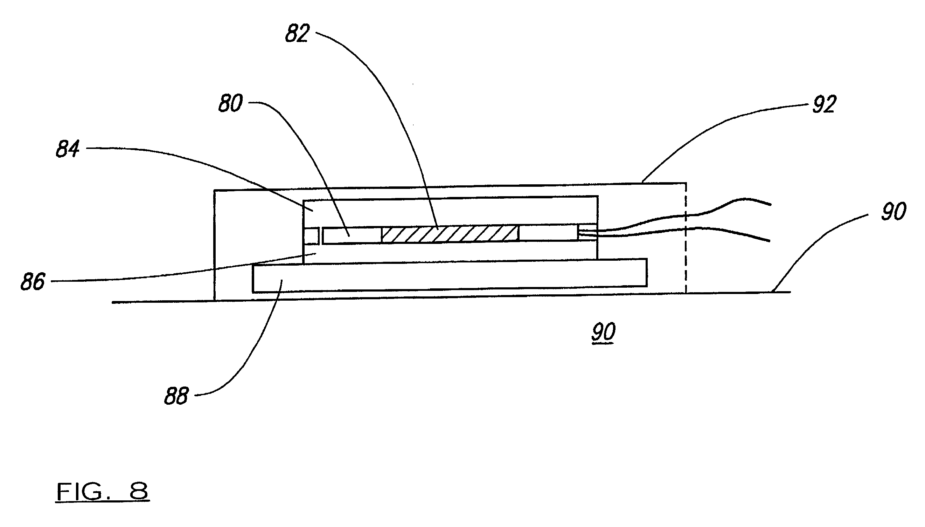

Referring finally to Figure 8, there is shown a schematic representation of the manner in which the transducer is embedded in a gel-based composition. The transducer 80 having a movable diaphragm 82 is sandwiched between two layers 84, 86 of a gel-based composition which contact the diaphragm 82. The excitation of the diaphragm 82 by the drive electronics mentioned above therefore transmits a the combined high and low frequency pressure wave representative of foetal heart movements to the gel-based composition which has an acoustic damping and slight distortion effect on said pressure waves. The resulting pressure waves detected by the foetal heart monitor are more akin to foetal heart movements. It is to be stressed however that the distortion and damping effects of the gel-based composition are slight and serve only to reduce the particularly severe artefacts in the transmitted waveform. The overall shape of the waveform transmitted to the gel-based composition by the transducer is not significantly altered. It is desirable that the frequency response of the transducer be particularly good on account of the high frequency excitation signal which drives said transducer.

A layer 88 of a silicone insulator material is disposed between the lower layer 86 and the surface 90 on which the simulator rests to prevent pressure waves from the transducer travelling vertically downwardly from being reflected at the interface between the simulator and the surface 90 back towards the monitoring apparatus which is typically positioned on top of the simulator as shown in Figure 1. A cover 92, which may be a stretched fabric or plastics material is disposed around the gel and transducer arrangement, and additionally, this cover may be extended below the transducer and insulator layer to accommodate the drive electronics and optionally a battery or mains power connector.

## Claims

1. A foetal heart simulator comprising an electronically controllable movement transducer connected to signal processing and production means which is capable of reproducing a frequency spectrum similar to that exhibited by the heart of a foetus, **characterised in that** the transducer is embedded within a gel-based coupling medium enclosed within a casing.

2. A simulator according to claim 1 wherein layers of the gel-based coupling medium are disposed on both sides of the transducer.

3. A simulator according to claim 2 wherein the layers of gel-based coupling medium are disposed on both sides of the axis of said transducer which is perpendicular to the axis of physical excitation thereof.

4. A simulator according to any preceding claim wherein the signal processing and production means comprises an electronic control system.

5. A simulator according to any preceding claim wherein the gel-based coupling medium completely fills the inside of the casing such that at least an amount of coupling medium separates the transducer from the casing on all sides.

6. A simulator according to any preceding claim wherein the signal processing and production means is capable of producing an excitation waveform to drive the transducer which is representative of the physiological response of a foetal heart.

7. A simulator according to any preceding claim wherein the signal processing and production means incorporates signal summation means and separate signal production means capable of producing a signals in which a low frequency component and a high frequency component are combined.

8. A simulator according to any of claims 1-6 wherein the signal processing and production means provides an output signal including both a high frequency and a low frequency component.

9. A simulator according to any one of claims 1-6 wherein the signal processing and production means provides an output signal in the form of an amplitude modulated envelope signal.

10. A simulator according to any preceding claim wherein the signal processing and production means includes a processor with associated memory in which is stored digital information from which a variety of different waveforms can be reconstructed and output to the transducer at the selection of an operator of said simulator.

11. A simulator according to claim 11 wherein the processor is programmatically capable of combining two or more sets of digital information stored in memory, a first set of digital information being representative of foetal heart movements, the second and further sets being representative of a physiological phenomenon, for example arrhythmia, hypoxia, muscular difficulties or other condition which may cause distress in a foetus and thus affect the heart rate thereof.

12. A simulator according to any preceding claim wherein the transducer is a piezo-electric transducer.

## Patentansprüche

1. Fötusherzsimulator, umfassend einen elektronisch steuerbaren Bewegungsmesswandler, der an ein Signalverarbeitungs- und -erzeugungsmittel angeschlossen ist, das ein Frequenzspektrum reproduzieren kann, das dem eines Fötusherzens ähnlich ist, **dadurch gekennzeichnet, dass** der Messwandler in einem Kopplungsmedium auf Gelbasis eingebettet ist, das in einer Verkleidung enthalten ist.

2. Simulator nach Anspruch 1, wobei Lagen des Kopplungsmediums auf Gelbasis auf beiden Seiten des Messwandlers angeordnet sind.

3. Simulator nach Anspruch 2, wobei die Lagen aus Kopplungsmedium auf Gelbasis auf beiden Seiten der Achse des genannten Messwandlers angeordnet sind, die lotrecht zur Achse seiner physikalischen Anregung ist.

4. Simulator nach einem der vorherigen Ansprüche, wobei das Signalverarbeitungs- und -erzeugungsmittel ein elektronisches Steuersystem umfasst.

5. Simulator nach einem der vorherigen Ansprüche, wobei das Kopplungsmedium auf Gelbasis die Innenseite der genannten Verkleidung vollständig ausfüllt, so dass wenigstens eine Menge des Kopplungsmediums den Messwandler auf allen Seiten von der Verkleidung trennt.

6. Simulator nach einem der vorherigen Ansprüche, wobei das Signalverarbeitungs- und -erzeugungsmittel eine Anregungswellenform erzeugen kann, um den Messwandler anzutreiben, die für die physiologische Reaktion eines Fötusherzens repräsentativ ist.

7. Simulator nach einem der vorherigen Ansprüche, wobei das Signalverarbeitungs- und -erzeugungsmittel ein Signalsummiermittel und ein separates Signalerzeugungsmittel beinhaltet, das Signale erzeugen kann, in denen eine niederfrequente Komponente und eine hochfrequente Komponente kombiniert sind.

8. Simulator nach einem der Ansprüche 1-6, wobei das Signalverarbeitungs- und -erzeugungsmittel ein Ausgabesignal erzeugt, das eine eine niederfrequente Komponente und eine hochfrequente Komponente enthält.

9. Simulator nach einem der Ansprüche 1-6, wobei das Signalverarbeitungs- und -erzeugungsmittel ein Ausgabesignal in der Form eines amplitudenmodulierten Hüllsignals bereitstellt.

10. Simulator nach einem der vorherigen Ansprüche, wobei das Signalverarbeitungs- und -erzeugungsmittel einen Prozessor mit assoziiertem Speicher beinhaltet, in den digitale Informationen gespeichert werden, anhand derer auf eine Auswahl des Bedieners des genannten Simulators hin eine Vielfalt verschiedener Wellenformen rekonstruiert und an den Messwandler ausgegeben werden können.

11. Simulator nach Anspruch 11, wobei der Prozessor programmatisch zwei oder mehr Sätze digitaler Informationen, die im Speicher gespeichert sind, kombinieren kann, wobei ein erster Satz digitaler Informationen für Fötusherzbewegungen repräsentativ ist, der zweite und weitere Sätze für ein physiologisches Phänomen repräsentativ ist/sind, wie zum Beispiel Arrhythmie, Hypoxie, Muskelschwierigkeiten oder andere Zustände, die Distress in einem Fötus auslösen und somit seine Herzrate beeinflussen können.

12. Simulator nach einem der vorherigen Ansprüche, wobei der Messwandler ein piezoelektrischer Messwandler ist.

## Revendications

1. Un simulateur du coeur foetal comprenant un transducteur de mouvements à commande électronique, connecté à des moyens de traitement et de production de signaux, qui est capable de reproduire un spectre de fréquences semblable à celui que présente le coeur d'un foetus, **caractérisé en ce que** le transducteur est enrobé dans un milieu de couplage à base de gel renfermé dans un boîtier.

2. Un simulateur selon la revendication 1, dans lequel des couches du milieu de couplage à base de gel sont disposées sur les deux côtés du transducteur.

3. Un simulateur selon la revendication 2, dans lequel les couches de milieu de couplage à base de gel sont disposées des deux côtés de l'axe dudit transducteur qui est perpendiculaire à l'axe d'excitation physique de celui-ci.

4. Un simulateur selon l'une quelconque des revendications précédentes, dans lequel les moyens de traitement et de production de signaux comprennent un système de commande électronique.

5. Un simulateur selon l'une quelconque des revendications précédentes, dans lequel le milieu de couplage à base de gel remplit complètement l'intérieur du boîtier, de sorte qu'une certaine quantité au moins du milieu de couplage sépare le transducteur du boîtier de tous les côtés.

6. Un simulateur selon l'une quelconque des revendications précédentes, dans lequel les moyens de traitement et de production de signaux sont capables, pour attaquer le transducteur, de produire une forme d'onde d'excitation représentative de la réponse physiologique d'un coeur foetal.

7. Un simulateur selon l'une quelconque des revendications précédentes, dans lequel les moyens de traitement et de production de signaux comprennent des moyens de sommation de signaux et des moyens de production de signaux séparés capables de produire un signal dans lequel sont combinées une composante à fréquence basse et une composante à fréquence élevée.

8. Un simulateur selon l'une quelconque des revendications 1 à 6, dans lequel les moyens de traitement et de production de signaux fournissent un signal de sortie qui comprend tant une composante à fréquence élevée qu'une composante à fréquence basse.

9. Un simulateur selon l'une quelconque des revendications 1 à 6, dans lequel les moyens de traitement et de production de signaux fournissent un signal de sortie sous forme d'un signal à enveloppe modulée en amplitude.

10. Un simulateur selon l'une quelconque des revendications précédentes, dans lequel les moyens de traitement et de production de signaux comprennent un processeur, avec une mémoire associée dans laquelle sont mémorisées des informations numériques à partir desquelles il est possible de reconstruire plusieurs formes d'ondes différentes et de les transmettre au transducteur lorsqu'elles sont sélectionnées par un opérateur dudit simulateur.

11. Un simulateur selon la revendication 10, dans lequel le processeur est capable, par programmation, de combiner deux ensembles (ou davantage) d'informations numériques stockées en mémoire, dont un premier ensemble d'informations numériques est représentatif des mouvements du coeur du foetus, le deuxième et autres ensembles étant représentatifs d'un phénomène physiologique, tel qu'arythmie, hypoxie, troubles musculaires ou autres anomalies risquant de causer une souffrance foetale, et donc de se répercuter sur le rythme cardiaque du foetus.

12. Un simulateur selon l'une quelconque des revendications précédentes, dans lequel le transducteur est un transducteur piézoélectrique.
